Europäisches Patentamt

**European Patent Office**

**Office européen des brevets**

(19)

(11) Publication number: **0 067 658**
**A2**

(12)

# EUROPEAN PATENT APPLICATION

(21) Application number: **82302982.2**

(22) Date of filing: **09.06.82**

(51) Int. Cl.³: **C 08 K 5/21**
**A 61 K 7/00, A 61 K 9/06**

(30) Priority: **15.06.81 US 273957**

(43) Date of publication of application:
**22.12.82 Bulletin 82/51**

(84) Designated Contracting States:
**AT BE CH DE FR GB IT LI LU NL SE**

(71) Applicant: **ALCON LABORATORIES INC**
**6201 South Freeway**
**Fort Worth Texas(US)**

(72) Inventor: **Damani, Nalikant Chunilal**
**6805 Capilla Court**
**Fort Worth Texas(US)**

(74) Representative: **Allard, Susan Joyce et al,**
**BOULT, WADE & TENNANT 27 Furnival street**
**London EC4A 1PQ(GB)**

(54) Carboxyvinyl polymer urea gel compositions.

(57) This invention relates to gel compositions comprising a carboxyvinyl polymer and urea.

EP 0 067 658 A2

Croydon Printing Company Ltd.

-1-

## CARBOXYVINYL POLYMER UREA GEL COMPOSITIONS

This invention relates to urea containing compositions useful as skin moisturizers, emollients, and dermatological therapeutic vehicles. More particularly, this invention relates to gel compositions composed of a carboxyvinyl polymer and urea and a method for preparing such gel compositions wherein the compositions are dermatologically safe and provide unique, long lasting moisturizing emollient and therapeutic effects to the skin.

Carboxyvinyl polymers are known and used in a number of dermatologic and cosmetic applications. These polymers are sold under the registered trademark Carbopol by the B.F. Goodrich Chemical Company, Cleveland, Ohio under the designations such as Carbopol 934, Carbopol 940, and Carbopol 941. These polymers are supplied as fluffy white powders having extremely high molecular weight. They are in acid form, and when dispersed and neutralized in water or other solvents form gels.

The use of Carbopol resins which upon neutralization may be used to thicken, suspend, and emulsify is well established. Inorganic alkalies such as sodium hydroxide, potassium hydroxide, and ammonium hydroxide, and organic amines such as triethanolamine, diisopropanolamine, alanine and glycine are all known and established neutralizing agents used to produce mucilages, gels or to thicken Carbopol containing compositions. Depending upon the desired viscosity, the carboxyvinyl polymers typically are gelled in a aqueous systems with the concentration of the polymer generally in the range of about 0.1% to about 3% by weight with the system being neutralized to a pH of about 7 with, for example, a 10% sodium hydroxide solution which forms about 0.02% by weight of the gel composition.

The use of gelled Carbopol polymers in cosmetic and pharmaceutical applications also is known. Hand creams, lotions, hair creams, cold waving lotions, and pharmaceutical ointments and jellies all have been prepared using Carbopol resins. Again, however, inorganic alkalies or organic amines have been used as agents to neutralize and gel the resins. Heretofore, the latter external neutralizing agents for the resins have not necessarily had any dermatological or comestic value, or dermatologically, even may be contraindicated or undesirable.

It has been found that an unique gel results from a mixture of carboxylvinyl polymer and urea without the need for external neutralizing agents. The invention provides the carboxylvinyl polymer and urea gel compositions and method for the preparation of the compositions wherein the compositions not only provide products with improved moisturization capabilities by allowing intimate contact of urea with the skin, but the compositions also provide elegant, convenient and efficacious therapeutic and comestic urea products. Further, therapeutic agents including but not limited to anti-inflammatory agents such as triamcinolone acetonide; antiseptics such as 4-chloro meta cresole; antipruritics, such as crotamiton; and keratolylic agents such as vitamin A acid or retenoic acid may be added to and may be a part of the gels of the invention.

To prepare the compositions of the invention, a Carbopol suspension is first prepared by slowly sprinkling the powdered polymer into the vortex of a vigorously agitated solvent in an amount effective to result in a gel of a desired viscosity upon addition of urea to the Carbopol dispersion, the Carbopol resin being generally from about 0.1% to about 3% percent by weight of the final gel composition. The solvent may be any appropriate urea solvent which is compatible with Carbopol, the solvent generally being water soluble; and

-3-

depending upon the application of the resulting composition, the solvent may be water. Other examples of solvents are alcohols, glycols, glycerol, and ketones. After dispersion of the Carbopol resin in the solvent and after allowing foam to break, an effective amount of urea sufficient to gel the Carbopol and in sufficient amount for the therapeutic, or cosmetic purpose of the final composition, generally from about 1% by weight to about 50% by weight of the resulting composition, is added to the dispersion with vigorous mixing. Although the entire procedure can be carried out at ambient temperature, heat up to about 90°C may be applied to facilitate formation of the gel with the addition of urea to the dispersion. Preservatives should be added to the resulting gels because of a susceptibility of the gels to growth of microorganisms and viscosity reduction from ultraviolet radiation; hence, biocides such as methyl-p-hydroxybenzoate, propyl-p-hydroxybenzoate or p-chloro-m-cresol, and ultraviolet absorbers such as Cyasorb UV-284, which is a product of American Cyanamid Co., are added as needed. Ethylenediamine tetracetic acid (EDTA) may be added as a metal ion chelating agent.

The following examples are provided to illustrate the invention more fully; however, they should not be construed as limiting the scope of the invention, many variations of which are contemplated.

### EXAMPLE I
### Skin Moisturizing Clear Gel

1.5 grams of Carbopol 940 was slowly added into the vortex of 70.0 grams of stirred water until dispersed. After dispersion, 2.0 grams of propylene glycol, 0.2 grams of methyl p-hydroxy benzoate, which is a preservative, and 0.05 grams of perfume were added to the dispersion. Thereafter, 10.0 grams of urea was slowly added to the dispersion with agitation with water

-4-

then being added to bring the total weight of the composition to 100 grams and forming a clear skin moisturizing gel.

## EXAMPLE II

### Wart Treatment Gel

1.0 grams of Carbopol 941 was slowly added to 40 grams of water as described in Example I. After dispersion of the Carbopol into the water, 5.0 grams of glycerine, 0.05 grams of p-chloro-m-cresol, which is a preservative, and 0.2 grams of perfume were added to the dispersion. Thereafter, 40.0 grams of urea was slowly added to the dispersion with agitation and heating to about 45°C to about 50°C with water then being added to bring the weight of the gel to 100 grams and forming a wart treatment gel.

## EXAMPLE III

### Moisturizing Gel Shampoo

1.0 gram of Carbopol 934 was slowly added to 75.0 grams of water as described in Example I. After dispersion of the Carbopol into the water, 12.0 grams of sodium lauryl ether sulfate, 0.5 grams cyclomethicone, 0.2 grams of Dowcil-200, which is a preservative and a product of Dow Chemicals, 0.2 grams of perfume, and 0.001 grams of FD&C Blue #1 for color were added to the dispersion. Thereafter, 5.0 grams of urea was slowly added to the dispersion with agitation and heating to about 60°C to about 70°C with water being added to bring the weight of the gel to 100 grams and forming a moisturizing gel shampoo.

## EXAMPLE IV

### Antipruritic Pharmaceutical Emulsion

0.5 grams of Carbopol 940 was slowly added to 50.0 grams of water as described in Example I. After dispersion, the suspension of Carbopol was heated to about 75°C, into which 2.0 grams of allantoin and 0.15 gram of methyl-p-hydroxybenzoate were dissolved.

-5-

In a separate vessel, 2.0 grams of stearic acid, 3.0 grams of mineral oil, 2.0 grams of glyceryl monostearate, 1.8 grams of Polysorbate-60 and 0.08 gram of propyl-p-hydroxybenzoate, which is a preservative, were combined and heated to about 70°C.

Contents of the two vessels were than combined with agitation to form a thin, milky emulsion. The heat sources were removed to begin cooling with agitation. At about 40°C, 0.15 gram of perfume and 4.0 grams of urea were added. The resultant viscous emulsion was then cooled to ambient temperature with agitation with water being added to make 100 grams of antipruritic emulsion.

## EXAMPLE V

### Anti-inflammatory Corticosteroid Gel

1.2 grams of Carbopol 934 was slowly added to 70 grams of water as described in Example I. After dispersion of the Carbopol into the water, 0.5 grams of hydrocortisone dissolved in 25 grams of alcohol, 5.0 grams of glycerine, and 0.7 grams of benzyl alcohol, which is a preservative, were added to the dispersion. Thereafter, 10 grams of urea was slowly added to the dispersion with agitation with water being added to bring the total weight of the composition to 100 grams and forming an anti-inflammatory corticosteroid gel.

The following Examples were prepared to study the pH (acidity or alkalinity) of the basic Carbopol-Urea gels, using Carbopol-940. The gels of the Examples were prepared by slowly dispersing the Carbopol into water with agitation. Urea was added to the Carbopol suspension with mixing when gelling occured. Although the application of heat was not made for these illustrations, later experiments showed no significant influence on the properties of the gel, except that the dissolution process of urea is accelerated.

-6-

## EXAMPLE VI

| Carbopol-940 | 1.0 gram |
| Urea | 1.0 gram |
| Water | 98.0 grams |

The pH of the gel was 2.9. The viscosity of the gel was 6000 cps, and was measured by a Brookfield viscometer, model LVT, spindle T-bar D at 12 rpm.

## EXAMPLE VII

| Carbopol-940 | 1.0 gram |
| Urea | 3.0 grams |
| Water | 96.0 grams |

The pH of the gel was 2.96. The viscosity of the gel was 9100 cps, and was measured by the method described in Example I.

## EXAMPLE VIII

| Carbopol | 1.0 gram |
| Urea | 5.0 grams |
| Water | 94.0 grams |

The pH of the gel was 3.1. The viscosity of the gel was 12,500 cps and was measured by the method described in Example I.

## EXAMPLE IX

| Carbopol | 1.0 gram |
| Urea | 10.0 grams |
| Water | 89.0 grams |

The pH of the gel was 3.25. The viscosity of the gel was 119,000 cps and was measured by the method described in Example I, except that 1.5 rpm spindle speed was used because of the high viscosity of the gel.

Examples VI to IX and the results of our measurements therein show that the gel-viscosity or the thickness increases with the increase in urea concentration. The effect of urea, however, on the pH of the Carbopol appears insignificant. This is important because in most topical preparations it is highly desirable to gel Carbopol suspensions without increasing the pH of the system.

CLAIMS :

1. A composition comprising a solvent selected from the group consisting of water, water soluble solvents, and mixtures thereof; a carboxyvinyl polymer; and an amount of area effective to form a gel.

2. A compoistion as claimed in Claim 1 wherein the polymer comprises from 0.5 to 3 percent by weight of the gel.

3. A composition as claimed in Claim 1 or Claim 2 wherein the urea comprises from 1.0 percent to 50.0 percent by weight of the gel.

4. An aqueous gel comprising a high molecular weight carboxyvinyl polymer; and an amount of urea effective to form said gel.

5. An aqueous gel as claimed in Claim 4 wherein the high molecular weight polymer comprises from 0.5 percent to 3.0 percent by weight of the gel.

6. An aqueous gel as claimed in Claim 4 or Claim 5 wherein the urea comprises from 1.0 percent to 50.0 percent by weight of the gel.

7. A method for preparing a gel comprising mixing a carboxyvinyl polymer into a solvent selected from the group consisting of water, water soluble solvents, and mixtures thereof to form a dispersion and mixing urea into the dispersion to form the gel.

8. A method as claimed in Claim 7 wherein the polymer has a high molecular weight and comprises from 0.1 percent to 3 percent by weight of the gel.

9. A method as claimed in Claim 8 wherein the solvent is water.

10. A method as claimed in Claim 8 or Claim 9 wherein the urea comprises from 1 percent to 50 percent by weight of the gel.